# EUROPEAN PATENT APPLICATION

(11) **EP 1 285 743 A1**
(43) Date of publication of application: **26.02.2003**
(21) Application number: 02024586.6
(22) Date of filing: 23.01.1992
(51) Int. Cl.: B32B 9/04, B32B 25/04, A61L 31/00, B29C 41/14

(54) **Latex article**

(62) Divisional of application: 99103415.8
(71) Applicant: Beck, Robin Thill, Los Angeles, California 90049 (US)
(72) Inventor: Beck, Robin Thill, California 90049 (US); Solomons, Clive C., CO 80222 (US); Plunkett, Jerry D., Colorado 80210 (US); Smith, Clayton S., Colorado 80401 (US)
(74) Representative: Grünecker, Kinkeldey, Stockmair & Schwanhäusser Anwaltssozietät

(57) **Abstract**

The latex article may be in the form of a glove, condom, diaphragm, slipper, overshoe, sterile band, catheter, tubing, drape, gut opening, mouthpiece, nipple, intra gastric nasal tube, kidney shunt, dam for teeth, brace for teeth, sub-clavian vein and artery shunt or colostomy bag.

## Description

This invention relates to the manufacture of latex articles incorporating a biocidal material for preventing the transmission and dissemination of disease and is especially applicable to the manufacture of such articles as gloves and condoms.

Latex has long been used for the manufacture of gloves and condoms for the purpose of inhibiting the transmission of disease-producing microbes and other harmful agents. Both the chemical inertness and the physical density of cured latex make it difficult for molecules and microbes to pass through the structure of the latex material. Nevertheless, latex materials are known to possess imperfections in the form of pits, pores and holes which can facilitate the transmission of such microbes and harmful agents through the latex material.

The present invention seeks to provide a procedure by means of which it is possible to make a chemical barrier against the transmission of such microbes and other harmful agents through a membrane such as latex.

The present invention now provides a method of forming a chemical barrier against the transmission of disease-causing microbes and other harmful agents through a membrane such as latex. In the method of the invention, a mold or former is coated with a biocide/coagulant which is dried, then the former is dipped into liquid latex. In an alternative procedure, the former is initially dipped into liquid latex which is allowed to gel before being dipped into the biocide/coagulant. The entire coating on the former is then cured. Alternatively, the biocide may be sprayed or otherwise applied onto the former or the gelled latex.

The present invention concerns the forming of a chemical barrier against disease-causing microbes and other harmful agents through a membrane fashioned from natural latex or from a synthetic latex material such as those known as natural skin, solvent cast membranes, elastomers, and polymers formed by curing the material from a liquid state. For convenience, the preferred aspects of the invention will be described with reference to a latex material. The latex material may be fashioned as a glove, condom, diaphragm, slipper, overshoe, sterile band, catheter, tubing, diaphragm, drape, gut opening, mouthpiece, baby nipple, intragastric nasal tube, nasal gastric tube, kidney shunt, rubber dam for teeth, plastic brace for teeth, sub-clavian vein or artery shunt, colostomy bag or any other product. Normally such latex products will be adapted for use in juxtaposition to a person's or animal's skin.

As used in the context of the present application, the term "biocide" means a substance or material which renders the disease-producing characteristic or harm-causing characteristic of a microbe or harmful agent ineffective substantially upon contact or shortly after contact of the microbe or harmful agent with the biocide. Examples of suitable biocides are dextran sulphate, nonoxynol-9, benzalkonium, betadyne, gentian violet, acriflavine and acridine dyes, mercurochrome, silver salts and an extract of blue green algae. Other examples of suitable biocides are set out in the list below.

Latex products, such as latex gloves and condoms, are conventionally formed by either a single dip or a double dip process. In these processes, a mold or former in the shape of the desired latex product is initially covered with a chemical coagulant, which allows a relatively thick, uniform, continuous layer of latex to be deposited on the former. The coagulant is dried on the former, and then the former is dipped into a vat of liquid latex for an appropriate period of time, whereby a film of latex is picked up on the surface of the former. The latex is allowed to wet gel into a sticky or tacky state and while in the gel state, is leached in a warm water rinse or bath. The warm water removes the coagulant as well as any residual ammonia or potassium hydroxide which might be present. In order to increase the thickness of latex, the process may be repeated so that a second film of latex is built up integrally over the first layer of latex. Usually the second layer is less thick than the first layer.

In accordance with the invention, the coagulant is selected so as to have biocidal properties so that the final latex product incorporates a biocide barrier. The method of the invention permits such latex products to be made by the general procedure described above and by the alternative procedure of initially applying a first liquid latex coating to the mold or former and then applying the biocide/coagulant to that first liquid latex coating before applying one or more further liquid latex coatings over the biocide/coagulant.

A biocide barrier can be incorporated into a latex product in the above manner, as follows. After the first dip of the former into the liquid latex and after the preferred warm water leach in a conventional manner, the former with the liquid latex in a wet gel state is dipped into a 0.10 to 5% by weight solution of gentian violet, for example a 0.33% by weight solution in water. The biocide solution coats the liquid latex. Alternatively, the biocide solution may be sprayed or otherwise applied to the wet gel latex. The biocide solution coating is then dried. Thereafter, the former is dipped again into a vat of liquid latex, may be leached again, and then the entire coating on the former is cured by drying.

It appears that the biocide solution acts as a coagulant, and causes the second layer of latex to be relatively thicker than is normally achieved without any biocide solution.

The amount of biocide solution applied either to the former or to the initial latex coating on the former is preferably such that the weight ratio of that biocide solution coating to the subsequent latex coating is 0.05 to 0.3.

A solution of 1.5% by weight of for example, gentian violet in water has also been used in this process, with slightly less desirable uniformity in thickness of the coatings. Nevertheless, it is believed that concentrations of up to about 5% by weight of gentian violet in water or in water and alcohol with or without other cosolvents can be advantageously used in the process.

It has been observed that the maximum amount of biocide solution picked-up by the wet gel latex coating the former is about 0.2 grams of solution per gram of latex.

It is believed that the biocide is bonded to and is permanently diffused within the surface of the latex, thereby substantially filling the pores and other imperfections of the latex.

The following biocides are believed to be equally suitable to those described above for use in the invention:-
halogen and halogen compounds - chlorine and iodine;
phenolic compounds;
alcohols;
oxidants;
chlorhexidine;
nitrogen compounds;
surface active agents, such as
   quaternary ammonium compounds,
   acid anionic compounds,
   amphoteric compounds,
   heavy metals and
   dyes, e.g. crystal violet;
antibiotics;
HC BLUE NO. 2
   (N¹,N⁴,N⁴,-(2-hydroxyethyl)-2-nitro-p-phenylenediamine);
NONOXYNOL-2
   (polyoxyethylene (2) nonyl phenyl ether);
   NONOXYNOL-4 and -8;
D & C RED NO. 22
   (eosine YS);
D & C RED NO. 37
   (rhodamine B-stearate);
D & C RED NO. 37 CALCIUM LAKE
   (rhodamine B stearate solvent);
and the following products listed according to their recommended or generally accepted biocidal applications:-

### Antimicrobial Soaps:

Cloflucarban
Para-chloro-mein-xylenof
Povidone-iodine complex
1.5 percent phenol or less aqueous/alcoholic
Triclocarbon
Tricloean

### Health-care Peronnel Handwash:

Benzalkonium chloride
Benzethonium chloride
Clofluearban
Hexylesorinal
Iodine complexed with phophate eater of alkyaryloxy polyethlene glycol
Methyl-benzethonium chloride
Nonyl phenoxypoly (ethyleneoxy) ethanol-iodine
Para-chloro-meta-xylenol
Povidene-iodine complex
1.5 percent phenol or less aqueous/alcoholic
Poloxamer-iodine complex
Tricloearban¹
Undecoylium chloride-iodine complex

### Patient preoperative skin preparation

Bonzalkonium chloride
Benzethonium chloride
Hexylresorcinoi
Iodine complexed with phosphate ester of alkylaryloxy polyethylene glycol
Methylbenxethonium chloride
Nonyl phenoxypoly (ethyleneoxy) ethanoilodine
Para-thloro-meta-xylenol
1.5 percent phenol or less aqueous/alcoholic
Poloxamer-iodine complex
Povidene-iodine complex
Undecoylium chloride-iodine complex

### Skin antiseptic

Benzalkonium chloride
Benzathonium chloride
Hexylresorcinoi
Iodine complexed with phosphate ester of alkylaryloxy polyethylene glycol
Iodine tincture
Methyl-bonzethonium chloride
Nonyl phenoxypoly (ethylencoxy) ethanoliodine
Para-chloro-meta-xylenol
1.5 percent phenol or less aqueous/alcoholic
Poloxamer-iodine complex
Povidene-iodine complex
Triclosan
Triple Dye
Undecoylium chloride-iodine complex

### Skin wound cleanser

Cloflutarban¹
Iodine complexed with phosphate ester of alkylaryloxy polyethylene glycol
Iodine tincture
Nonyl phenoxypoly (ethyleneoxy) ethanoliodine
Para-chloro-meta-xylenol
1.5 percent phenol or less aqueous/alcoholic
Poloxamer-iodine complex
Povidene-iodine complex
Tricloearban¹
Triclosan
Undecoylium chloride-iodine complex

### Skin wound protectant

Benzalkonium chloride
Benzathonium chloride
Hexylresorcinoi
Iodine complexed with phosphate ester of alkylaryloxy polyethylene glycol
Iodine tincture
Methyl-bonzethonium chloride
Nonyl phenoxypoly (ethylencoxy) ethanoliodine
Para-chloro-meta-xylenol

The present invention provides in particular a latex article having a biocide barrier, obtainable by a process comprising the steps of:
- applying onto a former a first coating consisting essentially of liquid latex and free of biocide;
- applying to the first coating of liquid latex a second coating consisting essentially of a biocide effective as a coagulant for liquid latex; and
- applying to said second coating a third coating consisting essentially of liquid latex and free of biocide.

Preferably in said process the third coating is thicker than the first coating and/or the first coating is leached with water prior to application of the second coating.

Preferably the second coating is dried before applying the third coating and/or said first coating is applied by dipping the former into liquid latex.

The second coating may be applied onto the first coating when the latex in the first coating is in a wet gel state.

Preferably the second coating is applied by spraying a biocide solution or said second coating is applied by dipping the former into a biocide solution. In any case, the biocide is applied preferably as a solution having a concentration of 0.10 to 5 percent by weight.

Preferably said third coating is applied by dipping the former in a vat of liquid latex.

Preferably the biocide is selected from chlorhexidine, dextran sulfate, triclosan, benzalkonium, betadyne, gentian violet, acriflavine and acridine dyes, mercurochrome, silver salts, and an extract of blue-green algae.

Preferably in said process the weight ratio of the biocide solution to the first latex coating is from 0.05 to 3 and/or the weight ratio of the biocide solution in the second coating to the latex applied in the third coating is between 0.05 and 0.3.

Preferably the biocide is bonded to and is permanently diffused within the surface of the latex membrane.

Preferably the latex article according to the present invention is in the form of a glove, condom, diaphragm, slipper, overshoe, sterile band, catheter, tubing, drape, gut opening, mouthpiece, nipple, intra gastric nasal tube, kidney shunt, dam for teeth, brace for teeth, sub-clavian vein and artery shunt or colostomy bag.
Preferably the liquid latex is selected among natural and synthetic latex and the synthetic latex is selected among solvent cast membranes, elastomers, polymers and synthetic latex known in the art as natural skin.

## Claims

1. A latex article having a biocide barrier, comprising:
a first coating consisting essentially of latex substantially free of biocide;
a second coating disposed on the first coating, the second coating consisting essentially of a biocide; and
a third coating disposed on the second coating, the third coating consisting essentially of latex substantially free of biocide.

2. The latex article according to claim 1, wherein the third coating is thicker than the first coating.

3. The latex article according to any of claims 1 or 2, wherein the biocide is a solution having a concentration of 0.10 to 5 percent by weight.

4. The latex article according to any of claims 1 to 3, wherein the biocide is selected from chlorhexidine, dextran sulfate, triclosan, benzalkonium, betadyne, gentian violet, acriflavine and acridine dyes, mercurochrome, silver salts, and an extract of blue-green algae.

5. The latex article according to any of claims 1 to 4, wherein the weight ratio of the biocide of the second coating to the latex of the first coating is from 0.05 to 3.

6. The latex article according to any of claims 1 to 5, wherein the weight ratio of the biocide of the second coating to the latex of the third coating is between 0.05 to 3.

7. The latex article according to any of claims 1 to 6, which is in the form of a glove, condom, diaphragm, slipper, overshoe, sterile band, catheter, tubing, drape, gut opening, mouthpiece, nipple, intra gastric nasal tube, kidney shunt, dam for teeth, brace for teeth, sub-clavian vein and artery shunt or colostomy bag.

8. The latex article according to any of claims 1 to 7, wherein the latex is selected from among natural and synthetic latex.

9. A latex article having a biocide barrier, comprising:
a first coating consisting essentially of a biocide; and
a second coating disposed on the first coating, the second coating consisting essentially of latex substantially free of biocide.

10. The latex article according to claim 9, wherein the biocide is a solution having a concentration of 0.10 to 5 percent by weight.

11. The latex article according to any of claims 9 to 10, wherein the biocide is selected from chlorhexidine, dextran sulfate, triclosan, benzalkonium, betadyne, gentian violet, acriflavine and acridine dyes, mercurochrome, silver salts, and an extract of blue-green algae.

12. The latex article according to any of claims 9 to 11, wherein the weight ratio of the biocide of the first coating to the latex of the second coating is from 0.05 to 3.

13. The latex article according to any of claims 9 to 12, which is in the form of a glove, condom, diaphragm, slipper, overshoe, sterile band, catheter, tubing, drape, gut opening, mouthpiece, nipple, intra gastric nasal tube, kidney shunt, dam for teeth, brace for teeth, sub-clavian vein and artery shunt or colostomy bag.

14. The latex article according to any of claims 9 to 13, wherein the latex is selected from among natural and synthetic latex.

15. An article having a biocide barrier, comprising:
a layer consisting essentially of natural skin; and
a coating disposed on the layer of natural skin, the coating consisting essentially of biocide.

16. The article according to claim 15, wherein the biocide is a solution having a concentration of 0.10 to 5 percent by weight

17. The article according to any of claims 15 to 16, wherein the biocide is selected from chlorhexidine, dextran sulfate, triclosan, benzalkonium, betadyne, gentian violet, acriflavine and acridine dyes, mercurochrome, silver salts, and an extract of blue-green algae.

18. The article according to any of claims 15 to 17, which is in the form of a glove, condom, diaphragm, slipper, overshoe, sterile band, catheter, tubing, drape, gut opening, mouthpiece, nipple, intra gastric nasal tube, kidney shunt, dam for teeth, brace for teeth, sub-clavian vein and artery shunt or colostomy bag.
